# EUROPEAN PATENT SPECIFICATION

(11) **EP 1 097 135 B1**
(45) Date of publication and mention of the grant of the patent: **07.05.2008**
(21) Application number: 99938233.6
(22) Date of filing: 13.07.1999
(51) Int. Cl.: C07D 209/42

(54) **PROCESS FOR THE PREPARATION OF AN INDOLE DERIVATIVE**
VERFAHREN ZUR HERSTELLUNG EINES INDOLDERIVATES
PROCEDE DE PREPARATION D'UN DERIVE D'INDOLE

(30) Priority: 16.07.1998 GB 9815483
(43) Date of publication of application: 09.05.2001
(73) Proprietor: SMITHKLINE BEECHAM PLC, Brentford, Middlesex TW8 9GS (GB)
(72) Inventor: FEDOULOFF, Michael, GlaxoSmithKline, Stavenage Hertfordshire, SG1 2NY (GB); STRACHAN, John Bryce, GlaxoSmithKline, Stavenage Hertfordshire, SG1 2NY (GB)
(74) Representative: Breen, Anthony Paul
(86) International application number: PCT/EP1999/004943
(87) International publication number: WO 2000/003983

(56) References cited:
- WO-A-98/07728

## Description

This invention relates to a new synthetic process to a compound having pharmacological activity.

WO 93/18036 (SmithKline Beecham plc) describes certain indole compounds having 5-HT₄ receptor antagonist activity including the compound of formula (I) 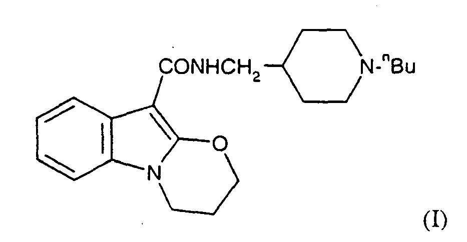 and its pharmaceutically acceptable salts. This compound is N-[(1-ⁿbutyl-4-piperidyl)methyl]-3,4-dihydro-2H-[1,3]oxazino[3,2-a]indole-10-carboxamide, referred to herein by its code number SB-207266, (the hydrochloride salt is SB-207266-A), which is being developed by SmithKline Beecham plc as the active ingredient in a medicament for treatment of irritable bowel syndrome.

Example 3 of WO 93/18036 describes a method of preparation of SB-207266-A from N-[(1-ⁿbutyl-4-piperidyl)methyl]indole-3-carboxamide (i.e. the compound corresponding to SB-207266, without the oxazino moiety), by reacting with N-chlorosuccinimide and 3-bromo-1-propanol, followed by treatment with sodium carbonate. N-[(1-ⁿbutyl-4-piperidyl)methyl]indole-3-carboxamide is prepared by coupling N-(1-ⁿbutyl-4-piperidyl)methylamine with a indole-3-carboxylic acid.

WO 98/07728 (SmithKline Beecham plc) describes a process for preparing SB-207266-A which involves the use of the N-(1-ⁿbutyl-4-piperidyl)methylamine intermediate at a later stage in the process thus resulting in an increased yield of SB-207266-A relative to the amount of this intermediate, which is relatively expensive to produce. In particular, the alternative process comprises the reaction of of N-(1-ⁿbutyl-4-piperidyl)methylamine with a compound of formula (A): 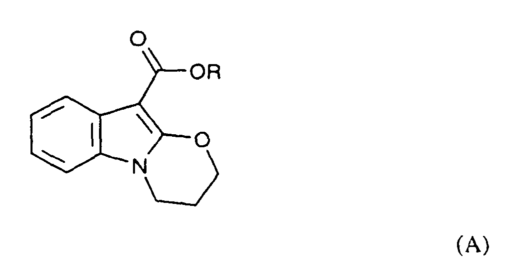 wherein R is alkyl, such as methyl or ethyl.

The compound of formula (A) wherein R is methyl is methyl 3,4-dihydro-2*H-*[1,3]-oxazino[3,2-a]indole-10-carboxylate.

WO98/07728 also describes the preparation of the oxazinoindole compound of the formula (A) from the corresponding indole by reaction with N-chlorosuccinimide and a 3-halo-propanol, such as 3-chloropropanol or 3-bromopropanol followed by cyclisation of the intermediate (B) by treatment with base in a suitable solvent.

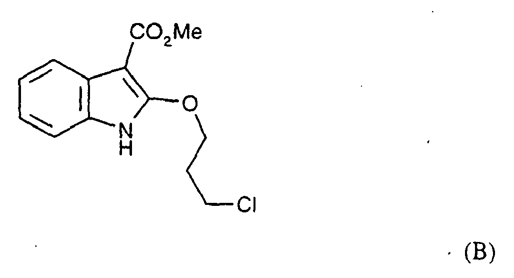

The Description in the latter specification describes in more detail the preparation of compound (B) from the corresponding methyl indole-3-carboxylate by reaction of the latter with N-chlorosuccinimide in the presence of 1,4-diazabicyclo[2.2.2]octane (DABCO) to form an intermediate of formula (C): 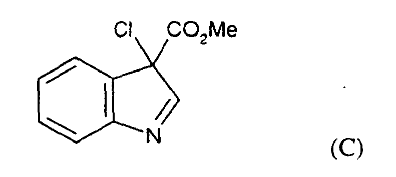 and subsequent reaction of (C) with 3-chloropropanol in the presence of methane sulphonic acid.

We have now found that the replacement of DABCO by a tertiary amine which is less nucleophilic than DABCO and which has a pK_{b} of from 8 to 11. especially 1,4-dimethylpiperazine, results in significant advantages for the commercial operation of the above reaction.

According to a feature of the present invention we provide a process for the preparation of the compound of formula (C) above, namely methyl 3-chloro-3H-indole-3-carboxylate, which comprises reacting a compound of formula (D) : 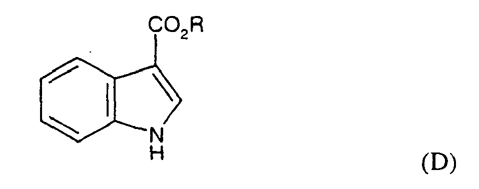 (in which R is methyl)
with N-chlorosuccinimide in the presence of a tertiary amine which is less nucleophilic than DABCO and which has a pK_{b} of from 8 to 11, especially 1,4-dimethylpiperazine (DMP).

Other examples of preferred bases for use in accordance with the invention are tetramethylethylenediamine and N-methylpiperidine.

The use of the above amine in place of DABCO has been found to increase significantly the overall yield of the process. The former amine also has the following advantages over DABCO:-
a) it is non-hygroscopic;
b) it does not react with dichloromethane, a preferred solvent for the reaction;
c) the product (C) is more stable allowing extended addition times and better temperature control;
d) quicker wash separations are possible during commercial manufacture; and
e) levels of the corresponding 2-methoxy compound, as an impurity, do not increase with extended process times.

The reaction is conveniently effected in an organic solvent such as dichloromethane or chloroform at a temperature in the range -20° C to +20° C. The resulting product of formula (C) can be used for the next step in the synthesis of SB-207266 e.g as described in WO 98/07728 without any need for isolation or purification.

The following Example illustrates the invention.

### Example

### Methyl 2-(3-chloropropoxy)-indole-3-carboxylate ( formula (B))

A mixture of methyl indole-3-carboxylate and dichloromethane is cooled to 0° C. 1,4-dimethylpiperazine (0.55eq.) and N-chlorosuccinimide (1.1 eq) are added and the mixture left to stir for two hours to give slurry containing the compound of formula (C) above. The resulting slurry is added to a solution of 3-chloropropanol (1.1 eq) and trichloroacetic acid (0.12 eq) in dichloromethane, maintaining the temperature below 0° C. The reaction mixture is left to stir for half an hour, then washed with 10% aqueous sodium carbonate, 0.5 M hydrochloric acid and water. The organic solution is dried over sodium sulphate, filtered and the solvent evaporated. Toluene is added and the mixture stirred at 0-5° C for one hour. The product is then filtered, washed with toluene and dried to give the title product in 83 % yield.

## Claims

1. A process for the preparation of methyl 3-chloro-3H-indole-3-carboxylate, namely the compound of formula (C): 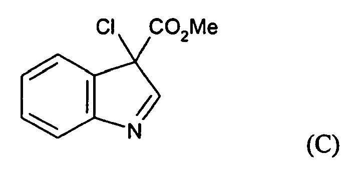 which comprises reacting a compound of formula (D) : 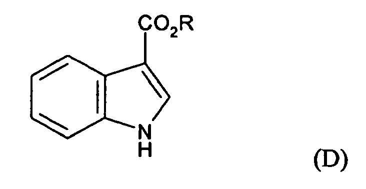 (in which R is methyl)
with N-chlorosuccinimide in the presence of a tertiary amine which is less nucleophilic than DABCO and which has a pK_{b} of from 8 to 11.

2. A process as claimed in claim 1 in which the amine is 1,4-dimethylpiperazine, tetramethylethylenediamine or N-methylpiperidine.

3. A process as claimed in claim 1 in which the amine is 1,4-dimethylpiperazine or tetramethylethylenediamine.

4. A process as claimed in claim 1 in which the amine is 1,4-dimethylpiperazine.

5. A process as claimed in any one of the preceding claims in which the reaction is effected in an organic solvent at a temperature in the range -20 °C to +20 °C.

6. A process as claimed in claim 5 wherein the organic solvent is dichloromethane or chloroform.

7. A process as claimed in any one of the preceding claims, wherein dichloromethane is used as a solvent for the reaction.

8. A process as claimed in any one of the preceding claims, wherein: a mixture of methyl indole-3-carboxylate and dichloromethane is cooled to 0 °C;
1,4-dimethylpiperazine (0.55 eq.) and N-chlorosuccinimide (1.1 eq.) are added; and the mixture is left to stir for two hours to give a slurry containing the compound of formula (C).

9. A process for preparing N-[(1-ⁿbutyl-4-piperidyl)methyl]-3,4-dihydro-2H-[1,3]oxazino[3,2-a]indole-10-carboxamide, i.e. the compound of Formula (I): 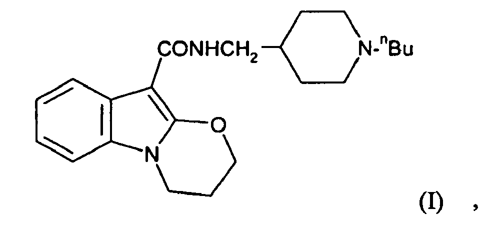 or a pharmaceutically acceptable salt thereof, comprising:
(a) preparing a compound of formula (C) according to a process as defined in any one of claims 1 to 8; and
(b) using the resulting compound of formula (C) in the next step in the synthesis of the N-[(1-ⁿbutyl-4-piperidyl)methyl]-3,4-dihydro-2H-[1,3]oxazino[3,2-a]indole-10-carboxamide or the pharmaceutically acceptable salt thereof.

10. A process as claimed in claim 9, wherein the compound of formula (C) is used without isolation or purification.

11. A process as claimed in claim 9 or 10 comprising, in step (b), preparing an oxazinoindole compound of formula (A): 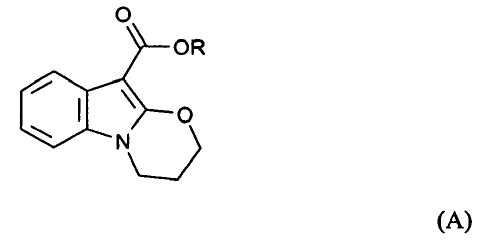 wherein R is methyl, by reaction of the compound of formula (C) with a 3-halo-propanol, followed by cyclisation of the resulting intermediate by treatment with base in a suitable solvent.

12. A process as claimed in claim 11 wherein the 3-halo-propanol is 3-chloropropanol or 3-bromopropanol.

13. A process as claimed in claim 9 or 10 comprising, in step (b), preparing an oxazinoindole compound of formula (A): 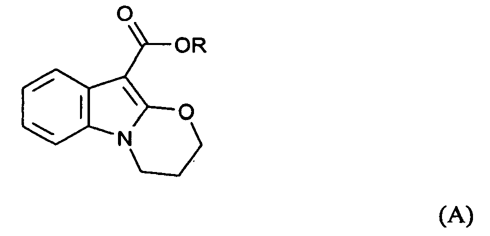 wherein R is methyl, by reaction of the compound of formula (C) with 3-chloropropanol, followed by cyclisation of the intermediate (B): 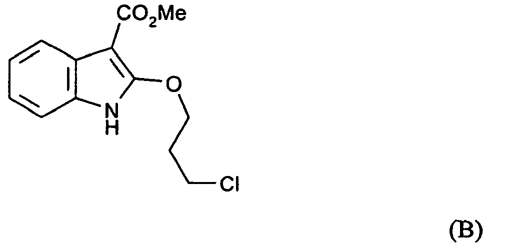 by treatment with base in a suitable solvent.

14. A process as claimed in claim 13 wherein:
a mixture of methyl indole-3-carboxylate and dichloromethane is cooled to 0 °C;
1,4-dimethylpiperazine (0.55eq.) and N-chlorosuccinimide (1.1 eq) are added and the mixture is left to stir for two hours to give a slurry containing the compound of formula (C);
the resulting slurry is added to a solution of 3-chloropropanol (1.1 eq) and trichloroacetic acid (0.12 eq) in dichloromethane, maintaining the temperature below 0° C;
the reaction mixture is left to stir for half an hour, then washed with 10% aqueous sodium carbonate, 0.5 M hydrochloric acid and water;
the organic solution is dried over sodium sulphate, filtered and the solvent is evaporated;
toluene is added and the mixture is stirred at 0-5° C for one hour;
and the product is then filtered, washed with toluene and dried to give methyl 2-(3-chloropropoxy)-indole-3-carboxylate of formula (B).

15. A process as claimed in any of claims 11 to 14, which is a process for preparing the hydrochloride salt of the N-[(1-ⁿbutyl-4-piperidyl)methyl]-3,4-dihydro-2H-[1,3]oxazino[3,2-a]indole-10-carboxamide (SB-207266-A), comprising reacting N-(1-ⁿbutyl-4-piperidyl)methylamine with the compound of formula (A).

16. A process as claimed in any of claims 9 to 14, which comprises preparing the hydrochloride salt of N-[(1-ⁿbutyl-4-piperidyl)methyl]-3,4-dihydro-2H-[1,3]oxazino[3,2-a]indole-10-carboxamide (SB-207266-A).

## Patentansprüche

1. Verfahren zur Herstellung von 3-Chlor-3H-indol-3-carbonsäuremethylester, nämlich die Verbindung der Formel (C): 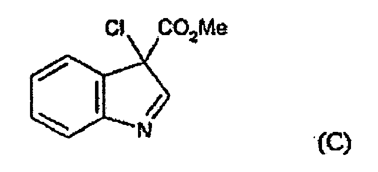 welches Umsetzen einer Verbindung der Formel (D): 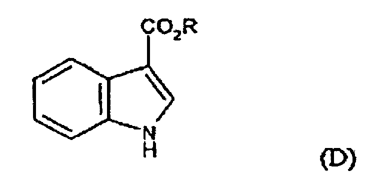 (wobei R Methyl ist) mit N-Chlorsuccinimid in Gegenwart eines tertiären Amins, welches weniger nukleophil als DABCO ist und welches einen pK_{b}-Wert von 8 bis 11 hat, umfasst.

2. Verfahren wie in Anspruch 1 beansprucht, wobei das Amin 1,4-Dimethylpiperazin, Tetramethylethylendiamin oder N-Methylpiperidin ist.

3. Verfahren wie in Anspruch 1 beansprucht, wobei das Amin 1,4-Dimethylpiperazin oder Tetramethylethylendiamin ist.

4. Verfahren wie in Anspruch 1 beansprucht, wobei das Amin 1,4-Dimethylpiperazin ist.

5. Verfahren wie in einem der vorstehenden Ansprüche beansprucht, wobei die Umsetzung in einem organischen Lösungsmittel bei einer Temperatur in einem Bereich von -20°C bis +20°C durchgeführt wird.

6. Verfahren wie in Anspruch 5 beansprucht, wobei das organische Lösungsmittel Dichlormethan oder Chloroform ist.

7. Verfahren wie in einem der vorstehenden Ansprüche beansprucht, wobei Dichlormethan als Lösungsmittel für die Umsetzung verwendet wird.

8. Verfahren wie in einem der vorstehenden Ansprüche beansprucht, wobei: ein Gemisch von Indol-3-carbonsäuremethylester und Dichlormethan auf 0°C gekühlt wird;
1,4-Dimethylpiperazin (0,55 Äq.) und N-Chlorsuccinimid (1,1 Äq.) zugegeben werden und das Gemisch für 2 Stunden gerührt wird, um eine Aufschlämmung zu ergeben, die die Verbindung der Formel (C) enthält.

9. Verfahren zur Herstellung von N-[(1-ⁿButyl-4-piperidyl)methyl]-3,4-dihydro-2H-[1,3]oxazino[3,2-a]indol-10-carboxamid, d.h. die Verbindung der Formel (I): 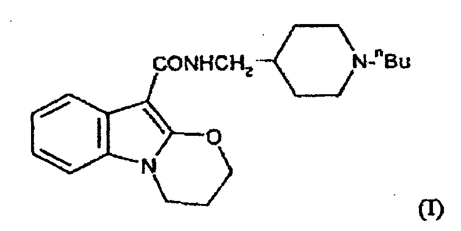 oder ein pharmazeutisch verträgliches Salz davon, umfassend:
(a) Herstellen einer Verbindung der Formel (C) gemäß einem Verfahren wie in einem der Ansprüche 1 bis 8 definiert; und
(b) Verwenden der resultierenden Verbindung der Formel (C) im nächsten Schritt der Synthese von N-[(1-ⁿButyl-4-piperidyl)methyl]-3,4-dihydro-2H-[1,3]oxazino[3,2-a]indol-10-carboxamid oder des pharmazeutisch verträglichen Salzes davon.

10. Verfahren wie in Anspruch 9 beansprucht, wobei die Verbindung der Formel (C) ohne Isolierung oder Reinigung verwendet wird.

11. Verfahren wie in Anspruch 9 oder 10 beansprucht, umfassend in Schritt (b) Herstellen einer Oxazinoindolverbindung der Formel (A): 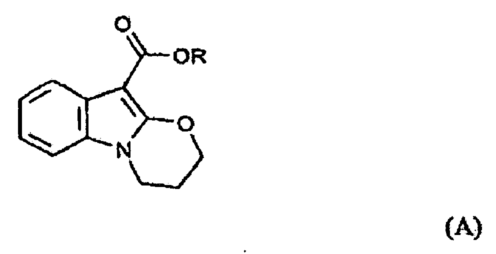 wobei R Methyl ist, durch Umsetzen der Verbindung der Formel (C) mit einem 3-Halogenpropanol, gefolgt vom Zyklisieren des resultierenden Zwischenprodukts durch Behandlung mit Base in einem geeigneten Lösungsmittel.

12. Verfahren wie in Anspruch 11 beansprucht, wobei das 3-Halogenpropanol 3-Chlorpropanol oder 3-Brompropanol ist.

13. Verfahren wie in Anspruch 9 oder 10 beansprucht, umfassend in Schritt (b) das Herstellen einer Oxazinoindolverbindung der Formel (A): 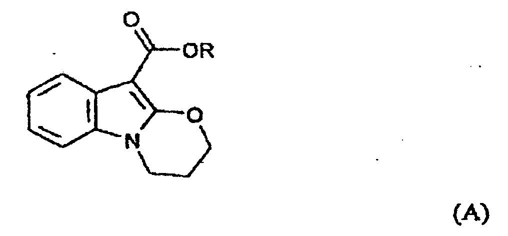 wobei R Methyl ist, durch Umsetzen der Verbindung der Formel (C) mit 3-Chlorpropanol, gefolgt vom Zyklisieren des Zwischenprodukts (B): 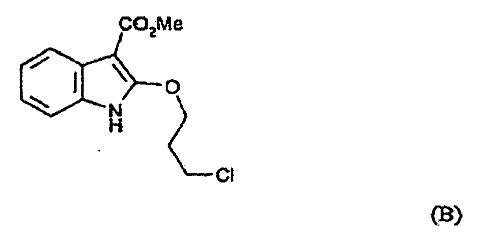 durch Behandlung mit Base in einem geeigneten Lösungsmittel.

14. Verfahren wie in Anspruch 13 beansprucht,
wobei: ein Gemisch von Indol-3-carbonsäuremethylester und Dichlormethan auf 0°C gekühlt wird;
1,4-Dimethylpiperazin (0,55 Äq.) und N-Chlorsuccinimid (1,1 Äq.) zugegeben werden und das Gemisch für 2 Stunden gerührt wird, um eine Aufschlämmung zu ergeben, die die Verbindung der Formel (C) enthält;
die resultierende Aufschlämmung zu einer Lösung von 3-Chlorpropanol (1,1 Äq.) und Trichloressigsäure (0,12 Äq.) in Dichlormethan zugegeben wird, wobei die Temperatur unter 0°C gehalten wird;
das Reaktionsgemisch eine halbe Stunde gerührt, dann mit 10 %iger wässriger Natriumcarbonatlösung, 0,5 M Salzsäure und Wasser gewaschen wird;
die organische Lösung über Natriumsulfat getrocknet, gefiltert und das Lösungsmittel verdampft wird;
Toluol zugegeben wird und das Gemisch für eine Stunde bei 0 bis 5°C gerührt wird;
und dann das Produkt gefiltert, mit Toluol gewaschen und getrocknet wird, um den 2-(3-Chlorpropoxy)-indol-3-carbonsäuremethylester der Formel (B) zu ergeben.

15. Verfahren wie in einem der Ansprüche 11 bis 14 beansprucht, welches ein Verfahren zur Herstellung des Hydrochloridsalzes von N-[(1-ⁿButyl-4-piperidyl)methyl]-3,4-dihydro-2H-[1,3]oxazino[3,2-a]indol-10-carboxamid (SB-207266-A) ist, umfassend Umsetzen von N-(1-ⁿButyl-4-piperidyl)methylamin mit der Verbindung der Formel (A).

16. Verfahren wie in einem der Ansprüche 9 bis 14 beansprucht, welches die Herstellung des Hydrochloridsalzes von N-[(1-ⁿButyl-4-piperidyl)methyl]-3,4-dihydro-2H-[1,3]oxazino[3,2-a]indol-10-carboxamid (SB-207266-A) umfasst.

## Revendications

1. Procédé pour la préparation de 3-chloro-3H-indole-3-carboxylate de méthyle, à savoir du composé de formule (C) : 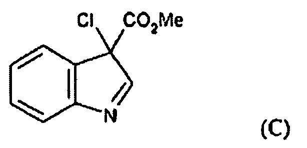 qui comprend la réaction d'un composé de formule (D) : 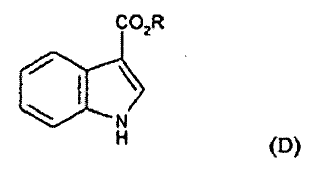 (dans laquelle R représente un groupe méthyle)
avec du N-chlorosuccinimide en présence d'une amine tertiaire qui est moins nucléophile que le DABCO et qui a un pK_{b} de 8 à 11.

2. Procédé suivant la revendication 1, dans lequel l'amine est la 1,4-diméthylpipérazine, la tétraméthyléthylènediamine ou la N-méthylpipéridine.

3. Procédé suivant la revendication 1, dans lequel l'amine est la 1,4-diméthylpipérazine ou la tétraméthyléthylènediamine.

4. Procédé suivant la revendication 1, dans lequel l'amine est la 1,4-diméthylpipérazine.

5. Procédé suivant l'une quelconque des revendications précédentes, dans lequel la réaction est conduite dans un solvant organique à une température comprise dans l'intervalle de -20°C à +20°C.

6. Procédé suivant la revendication 5, dans lequel le solvant organique est le dichlorométhane ou le chloroforme.

7. Procédé suivant l'une quelconque des revendications précédentes, dans lequel le dichlorométhane est utilisé comme solvant pour la réaction.

8. Procédé suivant l'une quelconque des revendications précédentes, dans lequel : un mélange d'indole-3-carboxylate de méthyle et de dichlorométhane est refroidi à 0°C ; de la 1,4-diméthylpipérazine (0,55 éq.) et du N-chlorosuccinimide (1,1 éq.) sont ajoutés ; et le mélange est laissé sous agitation pendant deux heures, ce qui donne une suspension contenant le composé de formule (C).

9. Procédé pour la préparation de N-[(1-ⁿbutyl-4-pipéridyl)méthyl]-3,4-dihydro-2H-[1,3]oxazino[3,2-a]indole-10-carboxamide, c'est-à-dire du composé de formule (I) : 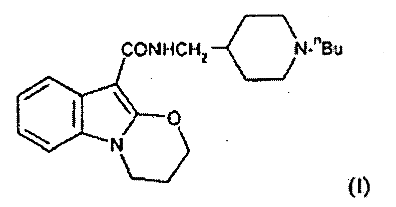 ou d'un de ses sels pharmaceutiquement acceptables, comprenant :
(a) la préparation d'un composé de formule (C) suivant un procédé tel que défini dans l'une quelconque des revendications 1 à 8 ; et
(b) l'utilisation du composé résultant de formule (C) dans l'étape suivante dans la synthèse du N-[(1-ⁿbutyl-4-pipéridyl)méthyl]-3,4-dihydro-2H-[1,3]oxazino[3,2-a]indole-10-carboxamide ou de son sel pharmaceutiquement acceptable.

10. Procédé suivant la revendication 9, dans lequel le composé de formule (C) est utilisé sans isolement ou purification.

11. Procédé suivant la revendication 9 ou 10, comprenant, dans l'étape (b), la préparation d'un dérivé d'oxazinoindole de formule (A): 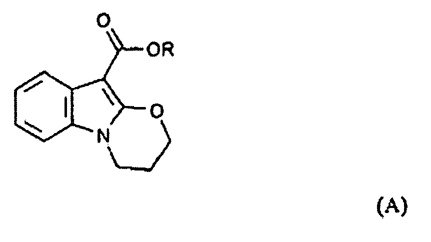 dans laquelle R représente un groupe méthyle, par réaction du composé de formule (C) avec un 3-halogénopropanol, puis par cyclisation de l'intermédiaire résultant par traitement avec une base dans un solvant convenable.

12. Procédé suivant la revendication 11, dans lequel le 3-halogénopropanol est le 3-chloropropanol ou le 3-bromopropanol.

13. Procédé suivant la revendication 9 ou 10, comprenant, dans l'étape (b), la préparation d'un dérivé d'oxazinoindole de formule (A): 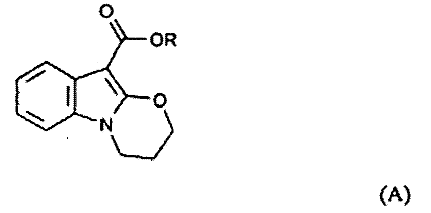 dans laquelle R représente un groupe méthyle, par réaction du composé de formule (C) avec du 3-chloropropanol, puis par cyclisation de l'intermédiaire (B) : 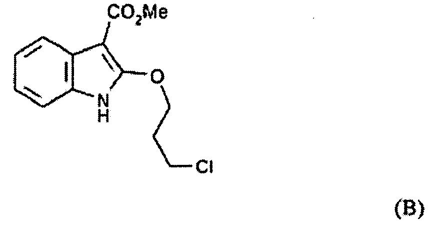 par traitement avec un base dans un solvant convenable.

14. Procédé suivant la revendication 13, dans lequel :
un mélange d'indole-3-carboxylate de méthyle et de dichlorométhane est refroidi à 0°C ;
de la 1,4-diméthylpipérazine (0,55 éq.) et du N-chlorosuccinimide (1,1 éq.) sont ajoutés ; et le mélange est laissé sous agitation pendant deux heures, ce qui donne une suspension contenant le composé de formule (C) ;
la suspension résultante est ajoutée à une solution de 3-chloropropanol (1,1 éq.) et d'acide trichloracétique (0,12 éq.) dans du dichlorométhane, en maintenant la température au-dessous de 0°C ;
le mélange réactionnel est laissé sous agitation pendant une demi-heure, puis est lavé avec une solution aqueuse à 10 % de carbonate de sodium, de l'acide chlorhydrique 0,5 M et de l'eau ;
la solution organique est déshydratée sur du sulfate de sodium, filtrée et le solvant est évaporé ;
du toluène est ajouté et le mélange est agité à une température de 0 à 5°C pendant une heure ;
puis le produit est filtré, lavé avec du toluène et séché, ce qui donne le 2-(3-chloropropoxy)-indole-3-carboxylate de méthyle de formule (B).

15. Procédé suivant l'une quelconque des revendications 11 à 14, qui est un procédé pour la préparation du chlorhydrate du N-[(1-ⁿbutyl-4-pipéridyl)méthyl]-3,4-dihydro-2H-[1,3]oxazino[3,2-a]indole-10-carboxamide (SB-207266-A), comprenant la réaction de N-(1-ⁿbutyl-4-pipéridyl)méthylamine avec le composé de formule (A).

16. Procédé suivant l'une quelconque des revendications 9 à 14, qui comprend la préparation du chlorhydrate du N-[(1-ⁿbutyl-4-pipéridyl)méthyl]-3,4-dihydro-2H-[1,3]oxazino-[3,2-a]indole-10-carboxamide (SB-207266-A).
